# EUROPEAN PATENT APPLICATION

(11) **EP 4 534 024 A1**
(43) Date of publication of application: **09.04.2025**
(21) Application number: 23201760.8
(22) Date of filing: 05.10.2023
(51) Int. Cl.: A61B 17/15, A61B 17/88

(54) **PATELLA CLAMPING DEVICE**

(71) Applicant: Aesculap AG, 78532 Tuttlingen (DE)
(72) Inventor: Snauwaert, Eliott, 52000 Chaumont (FR); Moussa, Said, 52000 Chamarandes-Choignes (FR)
(74) Representative: DREISS Patentanwälte PartG mbB

(57) **Abstract**

The invention relates to a patella clamping device (10), having a first clamping section (16) and a second clamping section (30), wherein the device comprises a sickle-shaped main body (12) having a distal end (14) which constitutes the first clamping section and having a proximal end (18) which includes a gripping section (20) to be gripped by the palm of a surgeon, the main body further having a pivot bearing (44) defining a pivot axis (46) of the second clamping section, wherein the second clamping section is pivotably movable with respect to the main body by means of an actuating mechanism (48) comprising a lever (50) to be activated by a finger of the surgeon such that clamping of a patella involves an initial engagement of the first clamping section with the patella and a subsequent movement of the second clamping section relative to the main body until engagement of the second clamping section with the patella.

## Description

The invention relates to a patella clamping device, having a first clamping section and a second clamping section, according to US 2006/0161165 A1.

As a part of a total knee replacement or total knee arthroplasty ("TKA") surgery, the posterior portion of a patella may be resected for implanting with a patellar knee component. The step of resecting the patella requires the use of a patella clamping device for retaining or holding the patella during cutting. US 2006/0161165 A1 proposes to provide a pincer-like structure with two mutually movable handle parts, one handle part being connected to a first clamping section engaging the skin / subcutaneous tissues overlying the anterior of a patella and the other handle part being connected to a second clamping section engaging the posterior of a patella, thus providing a clamping action along an antero-posterior axis of the patella.

One object of the present invention is to provide a patella clamping device which allows for a reliable clamping of a patella and for a comfortable handling by a surgeon.

This object is solved by the patella clamping device with the features of claim 1. Preferred embodiments are defined in the dependent claims.

According to the invention, a surgeon can predominantly handle a sickle-shaped main body which includes a gripping section to be gripped by the palm of a surgeon. This handling involves an initial engagement and positioning of the first clamping section with a first region of the patella while the second clamping section is its open position and still at a distance to second region of the patella. During the initial engagement and positioning, a surgeon can arrange the first clamping section in different positions relative to the patella, in a particular in different positions within a plane which is defined by the medio-lateral axis and the proximal-distal axis of the patella. Further, a surgeon may use his right or left hand in accordance with his preference.

Once the first clamping section is arranged in a final and preferable position in which the first clamping section engages with a first region of the patella, the second clamping section is moved - from its open position in relation to the first clamping action and into a clamping position - while the first clamping section at least essentially remains at its position. Movement of the second clamping section involves a pivoting movement with respect to the main body by means of an actuating mechanism. The actuating mechanism comprises a lever to be activated by a finger of the surgeon to cause a subsequent movement of the second clamping section relative to the main body until engagement of the second clamping section with a second region of the patella.

Since the surgeon can concentrate on each engagement of both clamping section in a subsequent manner, a patella can be clamped in a very reliable and comfortable manner.

According to a preferred embodiment, the first clamping section extends along an arc covering a clamping angle of at least 120°, preferably of at least 140°, in particular of at least 160°. These wide clamping angles provide for a shell-like structure surrounding a first portion of the patella, in particular a circumference of the patella which is situated in a plane defined by the medio-lateral axis and the proximal-distal axis of the patella.

According to a further preferred embodiment, the second clamping section extends along a straight axis. Thus, the second clamping section takes up little space only such that it does not interfere with the initial step of positioning of the first clamping section engaging the first patella portion. Once the second clamping section has been moved into its clamping position, it will be aligned in a tangential manner with a second portion of the patella and provide an essentially uniaxial pressing force. On other words: the clamping action of the second clamping section is free of lateral forces which could cause an involuntary change of the position and/or location of the patella.

According to a further preferred embodiment, the first clamping section and/or the second clamping section comprises or comprise a resection slot which is configured to insert a resection tool. In particular, such a resection slot is integrally built within the first clamping section and/or the second clamping section.

According to a particularly preferred embodiment, the first clamping section and the second clamping section define a clamping plane which is parallel with or coincides with a resection plane defined by at the at least one resection slot. This allows for providing a resection plane defined by the medio-lateral axis and the proximal-distal axis of the patella. The first clamping section and the second clamping sections can be arranged at any angle within such a plane; for example along the proximal-distal axis and along the medio-lateral axis and in any angular intermediate position. Further, the main body can be used in a first rotational position along a handling axis defined by the gripping section and in a second rotational position which is offset to the first rotational position by 180°.

For stably holding of a patella, the first clamping section and/or the second clamping section may comprise at least one row of clamping teeth. In particular, two parallel rows of teeth may be provided, the teeth of the rows being aligned or offset to each other.

In one embodiment, the main body comprises an additional pivot bearing defining a pivot axis of the lever. This allows for a very comfortable actuation of the lever by means of a finger of a surgeon.

In a further embodiment, the lever and the second clamping section are coupled to each other via at least one transmission element. This allows for a translation of movement from the lever to the second clamping section.

For example, the at least one transmission element may comprise a rotary sliding joint providing a simple and reliable link between the lever and the second clamping section, each pivoting about respective pivot axes.

In one embodiment, the actuating mechanism comprises a biasing element biasing the second clamping section in an open position and against a clamping position. The biasing element thus supports the initial positioning of the first clamping section without the need of a surgeon to manipulate the lever associated with the second clamping section.

In a preferred embodiment, the biasing element is a leaf spring providing a simple and reliable biasing of the lever.

According to a further preferred embodiment, the main body comprises an attachment mechanism for attaching an abutment element defining an abutment plane which is parallel a or the resection plane of the patella. Such an abutment element may be used to define and ensure a minimum thickness of the patella bone remaining after resection.

In one embodiment, the attachment mechanism comprises a linear guiding für guiding a stylus along an adjustment axis extending perpendicular to the resection plane, the stylus carrying the abutment element. To avoid any collision with the clamping sections, it is preferred that the linear guiding is positioned at a surface of the main body facing away from a clamping space of the clamping sections.

In a preferred embodiment, the linear guiding has two opposite openings facing away from each other for optional inserting the stylus into either one of the openings. This allows for the insertion and use of the stylus in two different / opposite directions.

Further features and advantages are the subject of the following description and of the diagrammatic illustration of embodiments.

In the drawings:
- Figure 1: shows a top perspective view of one embodiment of a patella clamping device, in an open position of a second clamping section;
- Figure 2: shows a rear perspective view of a detail of the patella clamping device, in a clamping position of the second clamping section; and
- Figure 3: shows a front view of the detail of the patella clamping device in clamping engagement with a patella.

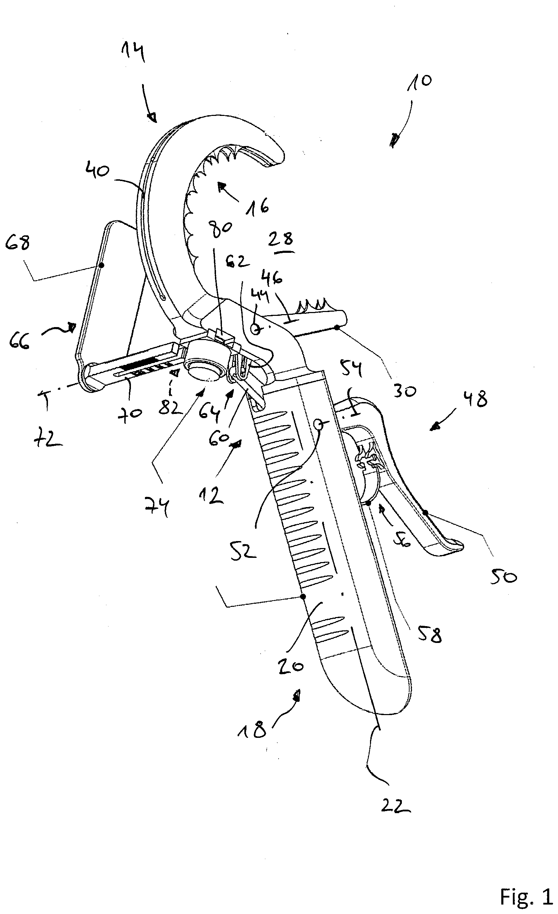

According to Fig. 1, a patella clamping device 10 comprises a sickle-shaped main body 12 having a distal end 14 which constitutes a first, arcuate clamping section 16 and having a proximal end 18 which includes a straight gripping section 20 to be gripped by the palm of a surgeon.

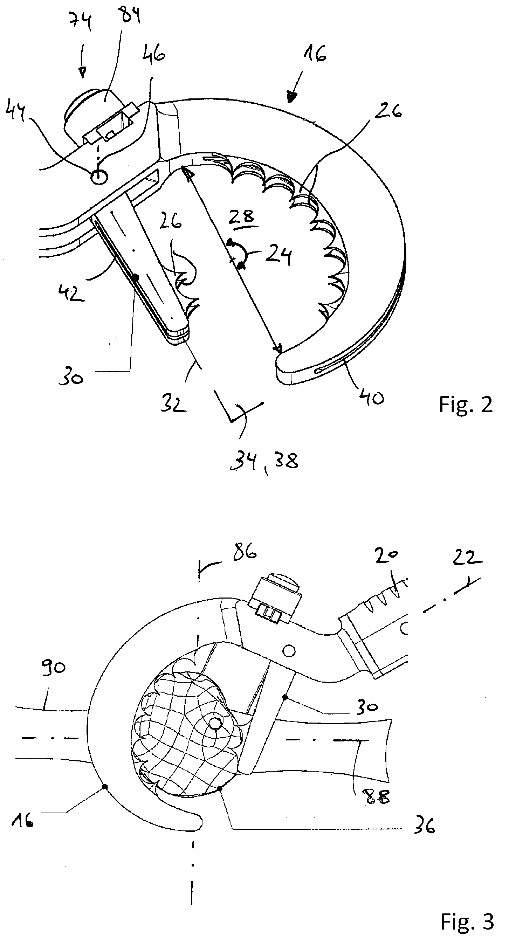

The gripping section 20 extends along a handling axis 22. The first clamping section 16 extends along an arc which covers a clamping angle 24 of e.g. 180°, see Fig. 2.

The first clamping section 16 comprises at least one row 26 of teeth which are directed to a clamping space 28 of the clamping device 10.

The clamping space 28 is delimited by the first clamping section 16 and a second clamping section 30. The second clamping section 30 comprises at least one row 26 of teeth which are directed to the clamping space 28 of the clamping device 10. Preferably, the second clamping section 30 extends along a straight axis 32.

The first clamping section 16 and the second clamping section 30 define a clamping plane 34 which is preferably parallel to or coincides with a resection plane 38 of a patella 36, see Fig. 3. The resection plane 38 is defined by a resection slot 40 of the first clamping section 16 and/or by a resection slot 42 of the second clamping section 30, see Fig. 2.

The main body 12 comprises a pivot bearing 44 defining a pivot axis 46 about which the second clamping section 30 is pivotably movable within the clamping plane 34. The pivot bearing 44 is located between the gripping section 20 and the first clamping section 16.

In order to control the position of the second clamping section 30 being an open position (see Fig. 1) or a clamping position (see Fig. 2 and 3), the patella clamping device comprises an actuating mechanism 48, see Fig. 1.

The actuating mechanism 48 comprises a lever 50 which is activated by a finger of the surgeon while the gripping section 20 is arranged in the palm of the surgeon. The lever 50 is pivotable about an additional pivot bearing 52 defining a pivot axis 54 of the lever 50. Preferably, the lever 50 is biased in a position corresponding to the open position of the second clamping section 30 by means of a biasing element 56 which in one embodiment may be a leaf spring 58.

Preferably, the pivot axes 46 and 54 are parallel to each other. The lever 50 comprises a driving section 60; the second clamping section 30 comprises a driven section 62. To transfer the movement of the lever 50 pivoting about its pivot axis 54 to the second clamping section 30 pivoting about its pivot axis 46, at least one transmission element is provided, for example a rotary sliding joint 64 movably connecting the driving section 60 to the driven section 62.

The patella clamping device 10 further comprises an abutment element 66 defining an abutment plane 68 which is parallel to the clamping plane 34 and/or to the resection plane 38. The abutment element 66 is connected to a stylus 70 extending along an adjustment axis 72 which is perpendicular to the clamping plane 34 and/or to the resection plane 38.

The patella clamping device 10 further comprises an attachment mechanism 74 for attaching the stylus 70 and the abutment element 66 to the main body 12. The attachment mechanism 74 comprises a linear guiding 76 for movably guiding the stylus 70 and the abutment element 66 along the adjustment axis 72.

The linear guiding 76 comprises two openings 80 and 82 facing away from each other to allow for the insertion of the stylus 70 into either one of the openings 80, 82 and in two opposite directions.

The attachment mechanism 74 further comprises a lock 84 for selectively releasing and locking the stylus 70 in a particular position along the adjustment axis 72.

Fig. 3 illustrates the clamping action of the patella clamping device 10 within a clamping plane 34 (parallel to the plane of the drawing) and a coinciding resection plane which is defined by a medio-lateral axis 86 and a proximal-distal axis 88 of the patella 36. The proximal-distal axis 88 is oriented in parallel with the main orientation of a patellar tendon 90.

In the embodiment shown, clamping action takes place along the proximal-distal axis 88. However, clamping action could take place at any angular position within the clamping plane 34 (360 degrees of freedom).

In addition, the patella clamping device 10 could be turned by 180° around the handling axis 22 and then also be used in any angular position within the clamping plane 34. Turning the patella clamping device 10 around the handling axis 22 is preferably accompanied by releasing the stylus 70 from one of the openings 80, 82 of the linear guiding 76 and inserting it into the other one of the openings 80, 82 prior to locking it again to the main body 12.

## Claims

1. Patella clamping device (10), having a first clamping section (16) and a second clamping section (30), **characterized in that** the device (10) comprises a sickle-shaped main body (12) having a distal end (14) which constitutes the first clamping section (16) and having a proximal end (18) which includes a gripping section (20) to be gripped by the palm of a surgeon, the main body (12) further having a pivot bearing (44) defining a pivot axis (46) of the second clamping section (30), wherein the second clamping section (30) is pivotably movable with respect to the main body (12) by means of an actuating mechanism (48) comprising a lever (50) to be activated by a finger of the surgeon such that clamping of a patella (36) involves an initial engagement of the first clamping section (16) with the patella (36) and a subsequent movement of the second clamping section (30) relative to the main body (12) until engagement of the second clamping section (30) with the patella (36).

2. Patella clamping device (10) according to claim 1, **characterized in that** the first clamping section (16) extends along an arc covering a clamping angle (24) of at least 120°, preferably of at least 140°, in particular of at least 160°.

3. Patella clamping device (10) according to one of the preceding claims, **characterized in that** the second clamping section (30) extends along a straight axis (32) .

4. Patella clamping device (10) according to one of the preceding claims, **characterized in that** the first clamping section (16) and/or the second clamping section (30) comprises or comprise a resection slot (40, 42) which is configured to insert a resection tool.

5. Patella clamping device (10) according to claim 4, **characterized in that in that** the first clamping section (16) and the second clamping section (30) define a clamping plane (34) which is parallel with or coincides with a resection plane (38) defined by the at least one resection slot (40, 42).

6. Patella clamping device (10) according to one of the preceding claims, **characterized in that** the first clamping section (16) and/or the second clamping section (30) comprises or comprise at least one row (26) of clamping teeth.

7. Patella clamping device (10) according to one of the preceding claims, **characterized in that** the main body (12) comprises an additional pivot bearing (52) defining a pivot axis (54) of the lever (50).

8. Patella clamping device (10) according to one of the preceding claims, **characterized in that** the lever (50) and the second clamping section (30) are coupled to each other via at least one transmission element.

9. Patella clamping device (10) according to claim 8, **characterized in that** the at least one transmission element comprises a rotary sliding joint (64).

10. Patella clamping device (10) according to one of the preceding claims, **characterized in that** the actuating mechanism (48) comprises a biasing element (56) biasing the second clamping section (30) in an open position and against a clamping position.

11. Patella clamping device (10) according to claim 10, **characterized in that** the biasing element (56) is a leaf spring (58).

12. Patella clamping device (10) according to one of the preceding claims, **characterized in that** the main body (12) comprises an attachment mechanism (74) for attaching an abutment element (66) defining an abutment plane (68) which is parallel to a or the resection plane (38) of the patella (36).

13. Patella clamping device (10) according to claim 12, **characterized in that** the attachment mechanism (74) comprises a linear guiding (76) for guiding a stylus (70) along an adjustment axis (72) extending perpendicular to the resection plane (38), the stylus (70) carrying the abutment element(66).

14. Patella clamping device (10) according to claim 13, **characterized in that** the linear guiding (76) has two opposite openings (80, 82) facing away from each other for optional inserting the stylus (70) into either one of the openings (80, 82).
